# EUROPEAN PATENT SPECIFICATION

(11) **EP 2 764 863 B1**
(45) Date of publication and mention of the grant of the patent: **11.03.2020**
(21) Application number: 12837822.1
(22) Date of filing: 03.10.2012
(51) Int. Cl.: A61K 31/137, A61K 31/472, A61P 25/28, A61P 43/00, A61K 31/198

(54) **TAU AGGREGATION INHIBITOR**
TAU-AGGREGATIONSHEMMER
INHIBITEUR D'AGRÉGATION DE PROTÉINES TAU

(30) Priority: 03.10.2011 JP 2011219059
(43) Date of publication of application: 13.08.2014
(73) Proprietor: National Center for Geriatrics and Gerontology, Obu-shi, Aichi 474-8511 (JP); The Doshisha, Kyoto 602-8580 (JP)
(72) Inventor: TAKASHIMA, Akihiko, Wako-shi Saitama 351-0104 (JP); SOEDA, Yoshiyuki, Shiki-shi Saitama 353-0004 (JP); OSADA, Hiroyuki, Nishitokyo-shi Tokyo 202-0004 (JP); IHARA, Yasuo, Kyotanabe-shi Kyoto 610-0394 (JP); MIYASAKA, Tomohiro, Kyotanabe-shi Kyoto 610-0394 (JP); SUGIMOTO, Hachiro, Kyotanabe-shi Kyoto 610-0394 (JP)
(74) Representative: Chevalier, Renaud Philippe
(86) International application number: PCT/JP2012/006363
(87) International publication number: WO 2013/051266

(56) References cited:
- WO-A2-01/78709
- WO-A2-03/020257
- WO-A2-2011/071920
- JP-A- 2003 530 432
- JP-A- 2003 532 634
- US-A1- 2010 167 286
- ANTHONY DACCACHE ET AL: "Oleuropein and derivatives from olives as Tau aggregation inhibitors", NEUROCHEMISTRY INTERNATIONAL, vol. 58, no. 6, 17 February 2011 (2011-02-17), pages 700-707, XP055000191, ISSN: 0197-0186, DOI: 10.1016/j.neuint.2011.02.010
- ALEX CROWE ET AL: "Identification of aminothienopyridazine inhibitors of Tau assembly by quantitative high-throughput screening", BIOCHEMISTRY, AMERICAN CHEMICAL SOCIETY, US, vol. 48, no. 32, 6 July 2009 (2009-07-06), pages 7732-7745, XP002691455, ISSN: 0006-2960, DOI: 10.1021/BI9006435 [retrieved on 2009-07-06]
- WOLOZIN B ET AL: "Olfactory neuroblasts from Alzheimer donors: Studies on APP processing and cell regulation", BIOLOGICAL PSYCHIATRY, ELSEVIER SCIENCE, NEW YORK, NY; US, vol. 34, no. 12, 15 December 1993 (1993-12-15), pages 824-838, XP024249588, ISSN: 0006-3223, DOI: 10.1016/0006-3223(93)90051-E [retrieved on 1993-12-15]
- WANG ET AL: "Compounds blocking mutant huntingtin toxicity identified using a Huntington's disease neuronal cell model", NEUROBIOLOGY OF DISEASE, BLACKWELL SCIENTIFIC PUBLICATIONS, OXFORD, GB, vol. 20, no. 2, 23 May 2005 (2005-05-23), pages 500-508, XP005118684, ISSN: 0969-9961, DOI: 10.1016/J.NBD.2005.03.026
- K. IQBAL ET AL: "Tau in Alzheimer Disease and Related Tauopathies", CURRENT ALZHEIMER RESEARCH, vol. 7, no. 8, December 2010 (2010-12), pages 656-664, XP055177832, United Arab Emirates ISSN: 1567-2050, DOI: 10.2174/156720510793611592
- AMBREE O ET AL: "Levodopa ameliorates learning and memory deficits in a murine model of Alzheimer's disease", NEUROBIOLOGY OF AGING, TARRYTOWN, NY, US, vol. 30, no. 8, 20 February 2008 (2008-02-20), pages 1192-1204, XP026184322, ISSN: 0197-4580, DOI: 10.1016/J.NEUROBIOLAGING.2007.11.010 [retrieved on 2008-02-20]
- J. LI ET AL: "Dopamine and L-dopa disaggregate amyloid fibrils: implications for Parkinson's and Alzheimer's disease", THE FASEB JOURNAL, vol. 18, no. 9, 1 April 2004 (2004-04-01), pages 962-964, XP055217613, ISSN: 0892-6638, DOI: 10.1096/fj.03-0770fje
- HUONG V T ET AL: "Catechol derivatives inhibit the fibril formation of amyloid-beta peptides", JOURNAL OF BIOSCIENCE AND BIOENGINEERING, ELSEVIER, AMSTERDAM, NL, vol. 109, no. 6, June 2010 (2010-06), pages 629-634, XP027051730, ISSN: 1389-1723 [retrieved on 2009-12-16]
- ONO K ET AL: "Anti-Parkinsonian agents have anti-amyloidogenic activity for Alzheimer's beta-amyloid fibrils in vitro", NEUROCHEMISTRY INTERNATIONAL, PERGAMON PRESS, OXFORD, GB, vol. 48, no. 4, 15 December 2005 (2005-12-15), pages 275-285, XP027957853, ISSN: 0197-0186 [retrieved on 2006-03-01]
- AIKEN CHARITY T ET AL: "A cell-based screen for drugs to treat Huntington's disease", NEUROBIOLOGY OF DISEASE, BLACKWELL SCIENTIFIC PUBLICATIONS, OXFORD, GB, vol. 16, no. 3, 14 May 2004 (2004-05-14), pages 546-555, XP009122873, ISSN: 0969-9961
- ANISH A KONKAR ET AL: "beta-Adrenoceptor Subtype Activities of Trimetoquinol Derivatives: Biochemical Studies on Human beta-Adrenoceptors Expressed in Chinese Hamster Ovary Cells 1", J PHARMACOL EXP THER., vol. 291, no. 2, November 1999 (1999-11), pages 875-883, XP055217896,
- PINDER R M ET AL: "HEXOPRENALINE A REVIEW OF ITS PHARMACOLOGICAL PROPERTIES AND THERAPEUTIC EFFICACY WITH PARTICULAR REFERENCE TO ASTHMA", DRUGS, ADIS INTERNATIONAL LTD, NZ, vol. 14, no. 1, 1977, pages 1-28, XP009186343, ISSN: 0012-6667

## Description

### TECHNICAL FIELD

The present invention relates to inhibitors for tau aggregation that causes neurologic deficits and synaptic losses.

### BACKGROUND ART

Alzheimer's disease (AD) is a type of dementia whose main symptoms are cognitive decline and personality change. Dementia is a common disorder, and about 25% of Japanese aged 85 years or older develop, and about a half of dementia cases are caused by AD. In Japan, there are about 1.6 to 1.8 million AD patients in 2011, and the number of AD patients has been increasing with aging of the population. This is a serious issue especially in Japan which has a decreasing birth rate and an aging population.

An acetylcholinesterase inhibitor that is considered as a most effective inhibitor for prevention and treatment of AD is effective only for patients with mild to moderate symptoms, and many researchers find no effectiveness of the acetylcholinesterase inhibitor for patients with advanced cases.

Although neuropathological findings on AD patients have two features: senile plaques of β-amyloid; and neurofibrillary tangles (NFTs) formed by abnormal accumulation of tau, a mainstream of current AD research is based on the amyloid-β hypothesis that abnormal accumulation of amyloid-β peptide eventually leads to AD.

However, it has been revealed that mutation of the tau gene promotes NFT formation and causes dementia in frontotemporal dementia and parkinsonism (FTDP) and that only aggregation and accumulation of tau in the brain cause neuronal abnormalities. Thus, the correlation between tau aggregation and AD occurrence has attracted attention in recent years.

Neuronal cells in CNS contain a large amount of tau, which is essential for the function of axons constituting the neural network of brain. Insoluble aggregation of tau in cells hinders axonal transport, leading to neuronal death.

PATENT DOCUMENT 1 describes a drug containing, as a main component, a naphthoquinone-type compound that inhibits tau aggregation for improving AD symptoms. This drug reduces tau aggregation in cells to some extent so that NFT formation is reduced and AD symptoms are alleviated.

### CITATION LIST

### PATENT DOCUMENT

[PATENT DOCUMENT 1] Japanese Unexamined Patent Publication (Japanese Translation of PCT Application) No. 2004-534854
Moreover, the publication of A. Daccache et al. entitled "Oleuropein and derivatives from olives as Tau aggregation inhibitors", Neurochemisty international, Vol. 58, n°6, 17 february 2011, pages 707-707 discloses the ability of three natural phenolic derivatives obtained from olives (namely hydroxytyrosol, oleuropein and oleuropein aglycone) to prevent such Tau fibrillization *in vitro.* They do inhibit Tau aggregation.
The publication of A. Crowe et al., entitled "Identification of aminothienopyridazine inhibitors of Tau assembly by quantitative high-throughput screening", Biochemistry, American Chemical Society, Vol 48, n°32, 6 July 2009, pages 7732-7745, discloses ATPZ molecules that are a novel class of tau assembly inhibitors.

### SUMMARY OF THE INVENTION

### TECHNICAL PROBLEM

The tau aggregation inhibitor described above, however, does not sufficiently inhibit tau aggregation in cells, and is insufficient for treatment of tauopathies including AD.

It is therefore an object of the present invention to provide a tau aggregation inhibitor that can sufficiently reduce tau aggregation in cells.

### SOLUTION TO THE PROBLEM

The present invention provides a tau aggregation inhibitor for use in prevention and/or treatment of tauopathy, wherein the tau aggregation inhibitor includes a catechol structure-containing compound or a salt thereof, and the catechol structure-containing compound is droxidopa; wherein the tauopathy excludes Alzheimer's disease.

### DISCLOSURE OF ANOTHER EXAMPLES OF TAU AGGREGATION INHIBITORS

Another examples of tau aggregation inhibitors are here below disclosed.
An example of a tau aggregation inhibitor includes isoprenaline or a salt thereof. Isoprenaline included in the tau aggregation inhibitor may be d-enantiomer. Alternatively, isoprenaline included in the tau aggregation inhibitor may be d/l-racemic mixture. It should be noted that d-enantiomer or d-isoprenaline refers to (S)-(+)-isoprenaline and d/1-racemic mixture or d/1-isoprenaline refers to a mixture of (S)-(+)-isoprenaline and (R)-(-)-isoprenaline.

Another examples of the tau aggregation inhibitors include a catechol structure-containing compound or a salt thereof, and the catechol structure-containing compound is selected from the group consisting of dopamine, dobutamine, levodopa, levodopa/carbidopa, trimetoquinol, hexoprenaline, methyldopa.

### ADVANTAGES OF THE INVENTION

According to the present invention, tau aggregation in cells can be sufficiently reduced. Thus, patients suffering from tauopathies excluding AD, for which no effective therapies have not been discovered yet, can be cured. In the current era of aging society, the technique disclosed herein can achieve more effective social contributions by, for example, improving quality of life in elderly population, alleviating the burden of cares, and reducing medical expenses.

### BRIEF DESCRIPTION OF THE DRAWINGS

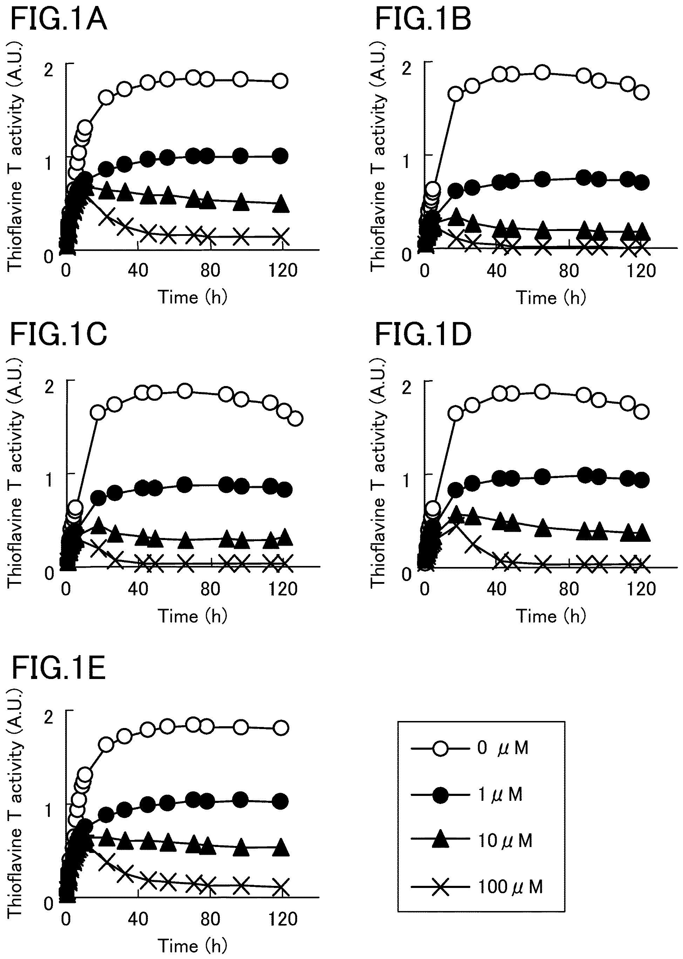

[FIG. 1] FIGS. 1A to IE show thioflavine T activity, FIG. 1A shows the effect of inhibiting thioflavine T activity by (R)-(-)-epinephrine, FIG. 1B shows the effect of inhibiting thioflavine T activity by levodopa, FIG. 1C shows the effect of inhibiting thioflavine T activity by dopamine, FIG. 1D shows the effect of inhibiting thioflavine T activity by norepinephrine, and FIG. IE shows the effect of inhibiting thioflavine T activity by isoprenaline.

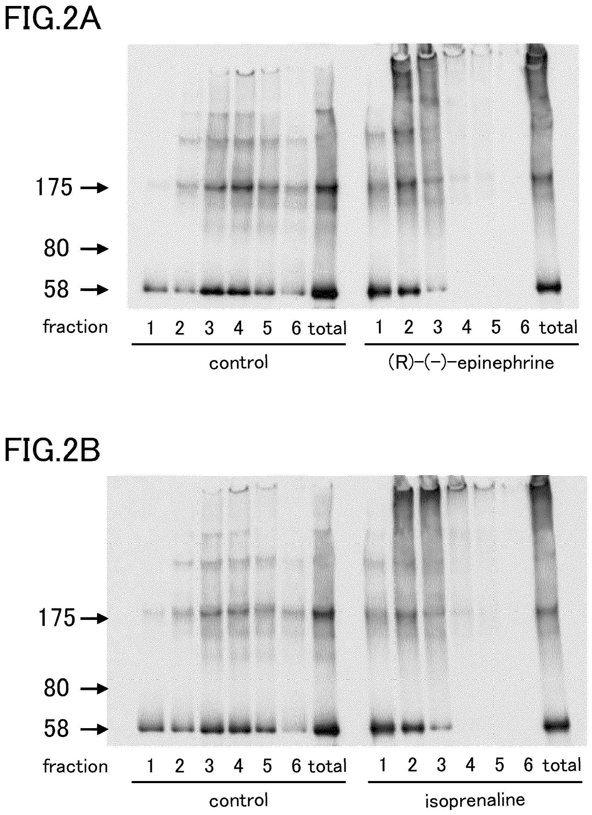

[FIG. 2] FIGS. 2A and 2B show results of sodium dodecyl sulfate (SDS)-PAGE western blotting indicating tau aggregation inhibition effects detected by sucrose-density gradient centrifugation, FIG. 2A shows results for (R)-(-)-epinephrine, and FIG. 2B shows results for isoprenaline.

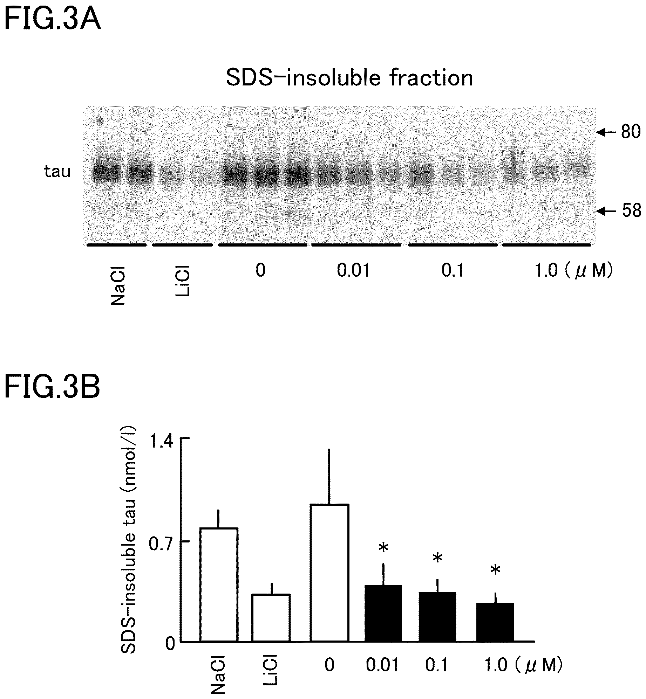

[FIG. 3] FIGS. 3A and 3B show that isoprenaline reduces SDS-insoluble tau aggregation, FIG. 3A shows detection of tau in an SDS-insoluble fraction, and FIG. 3B is a graph corresponding to FIG. 3A and shows that isoprenaline reduces the amount of SDS-insoluble tau.

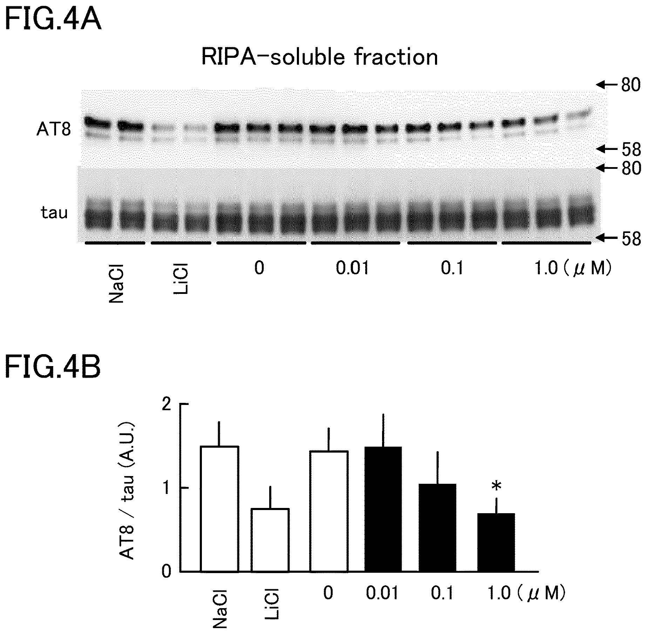

[FIG. 4] FIGS. 4A and 4B show the inhibition of tau phopsophorylation by isoprenaline, FIG. 4A shows detection of phosphorylated tau (AT8 site) and tau in an RIPA-soluble fraction, and FIG. 4B is a graph corresponding to FIG. 4A and shows that isoprenaline reduces the amount of phosphorylated tau in the RIPA-soluble fraction.

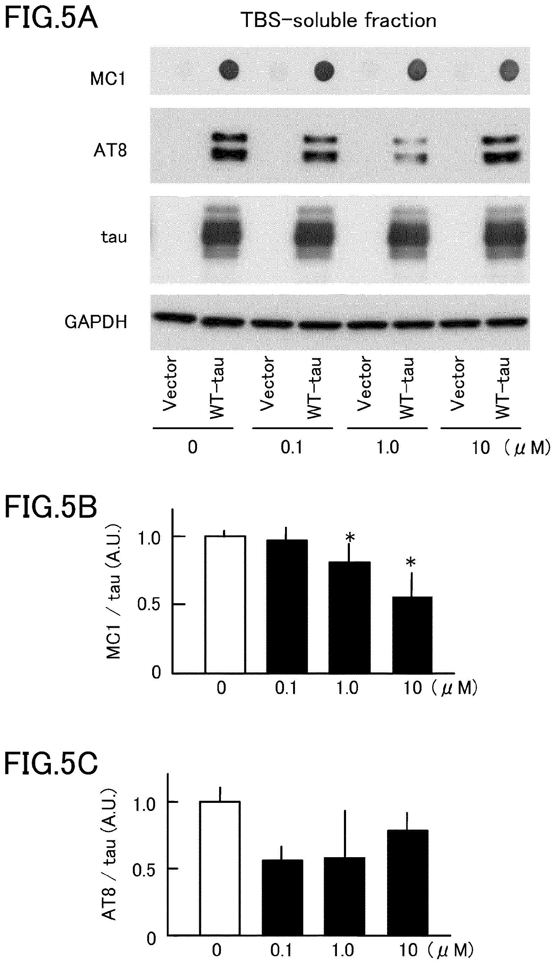

[FIG. 5] FIGS. 5A to 5C show effects of isoprenaline to a tau conformational change (that can be detected with MC1 antibody) observed in AD brain, FIG. 5A shows detection of MC1 antibody-labeled tau, tau phosphorylation (AT8 site), total tau, and GAPDH (loading control) in a TBS-soluble fraction, FIG. 5B shows the proportion of MC1 antibody-labeled tau to total tau, and FIG. 5C shows the proportion of phosphorylated tau to total tau.

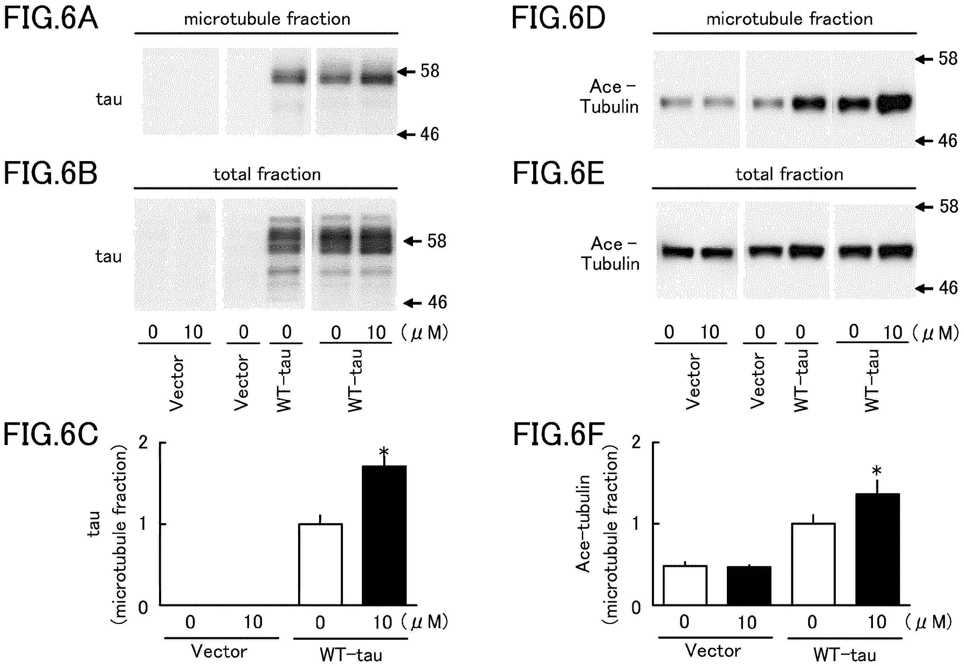

[FIG. 6] FIGS. 6A to 6F show increases in amount of tau and acetylated tubulin in microtubule fractions of isoprenaline, FIG. 6A shows detection of tau in a microtubule fraction, FIG. 6B shows detection of tau in a total fraction, FIG. 6C is a graph corresponding to FIG. 6A and shows that isoprenaline increases the amount of tau in the microtubule fraction, FIG. 6D shows detection of acetylated tubulin in a microtubule fraction, FIG. 6E shows detection of acetylated tubulin in a total fraction, FIG. 6F is a graph corresponding to FIG. 6D and shows that isoprenaline increases the amount of acetylated tubulin in the microtubule fraction.

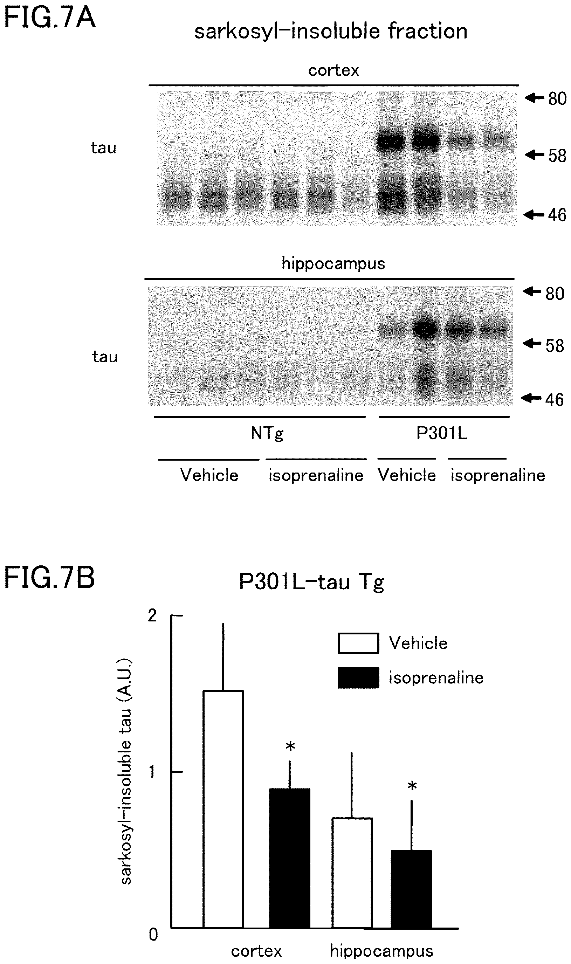

[FIG. 7] FIGS. 7A and 7B show decreases in amount of sarkosyl-insoluble tau in the brains of isoprenaline administered mice (P301L tau Tg mice), which overexpressing human P301L mutant tau, FIG. 7A shows detection of a sarkosyl-insoluble fraction, and FIG. 7B is a graph corresponding to FIG. 7A and shows that isoprenaline decreases the amount of sarkosyl-insoluble tau in the brains of P301L tau Tg mice.

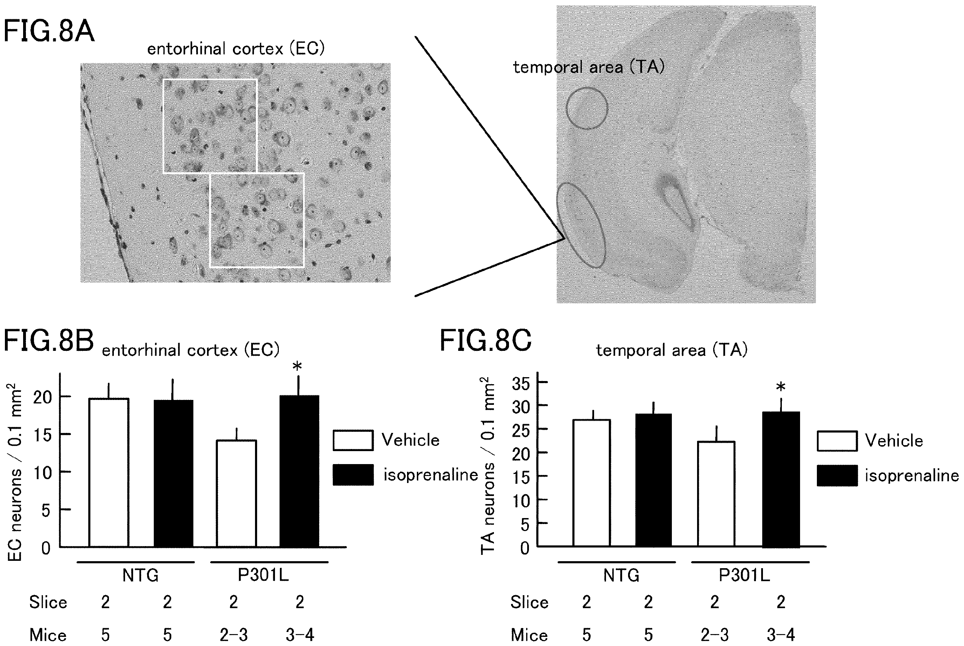

[FIG. 8] FIGS. 8A to 8C show that isoprenaline inhibits a decrease in the numbers of neurons in P301L tau Tg mice, FIG. 8A shows a brain region in which the number of neurons was counted and also shows a method for counting the number, FIG. 8B shows that isoprenaline inhibited a decrease in number of cells of the entorhinal cortex, and FIG. 8C shows that isoprenaline inhibited a decrease in number of cells in the temporal cortex.

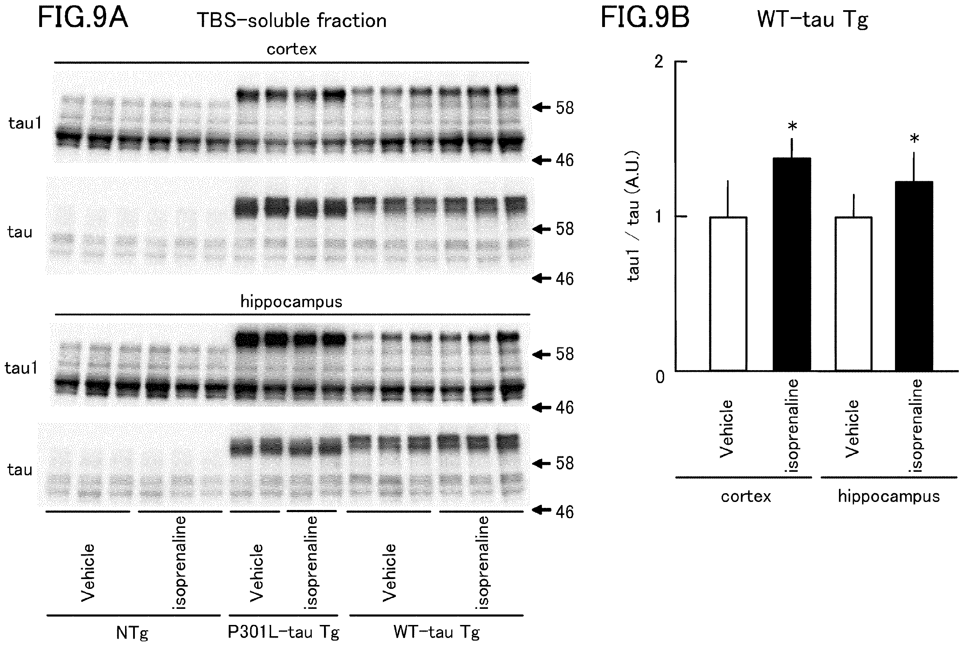

[FIG. 9] FIGS. 9A and 9B show an increase in dephosphorylation of TBS-soluble tau by isoprenaline in mice (WT tau Tg mice) overexpressing wild-type tau, FIG. 9A shows detection of dephosphorylation in a TBS-soluble fraction with tau1 antibody, and FIG. 9B shows the proportion of dephosphorylated tau to total tau.

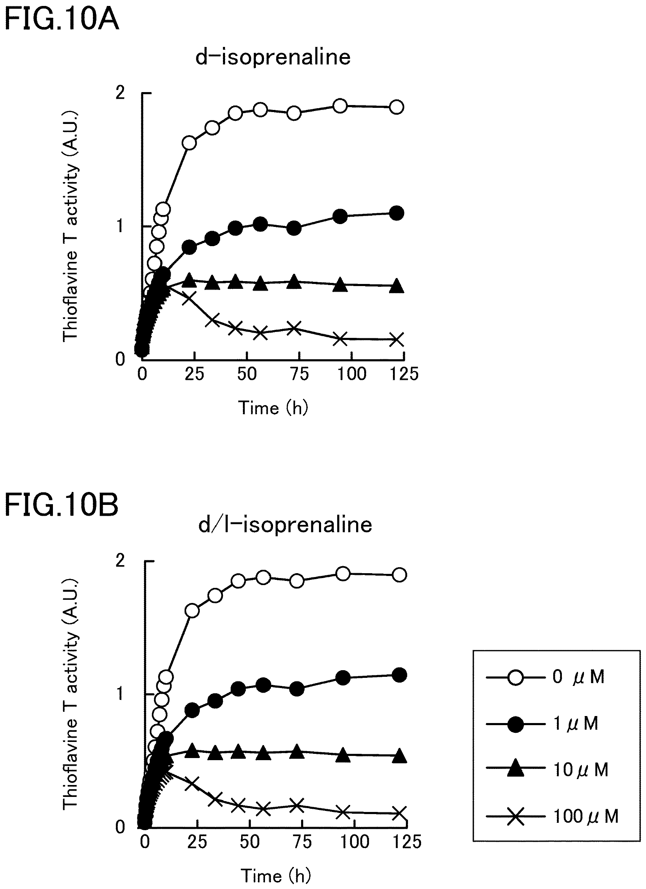

[FIG. 10] FIGS. 10A and 10B show a change in thioflavine T activity by d-isoprenaline, FIG. 10A shows the effect of inhibiting thioflavine T activity by d-isoprenaline, and FIG. 10B shows the effect of inhibiting thioflavine T activity by d/l-isoprenaline.

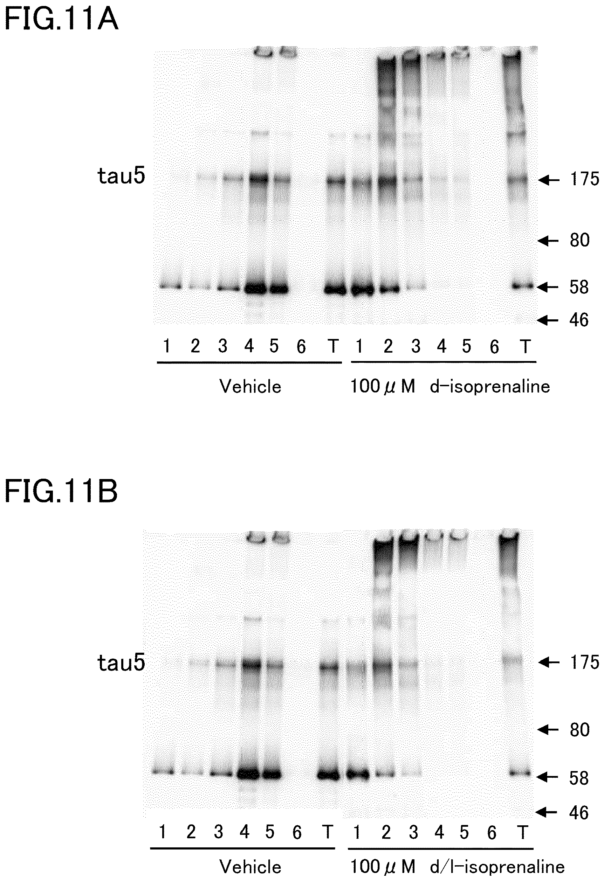

[FIG. 11] FIGS. 11A and 11B show results of SDS-PAGE western blotting indicating that inhibition of tau aggregation by d-isoprenaline detected by sucrose-density gradient centrifugation, FIG. 11A shows results for d-isoprenaline, and FIG. 11B shows results for d/1-isoprenaline.

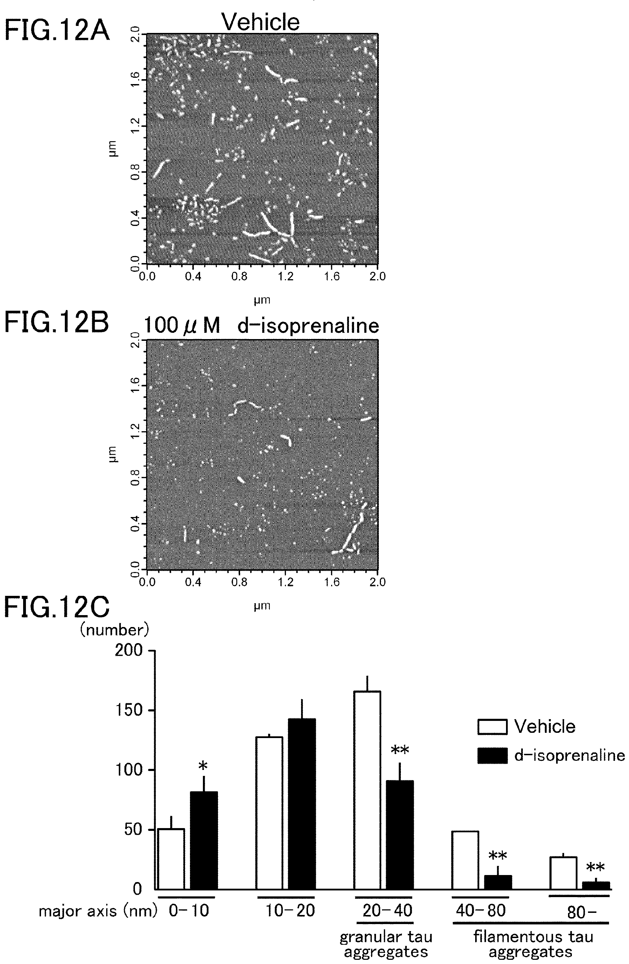

[FIG. 12] FIGS. 12A to 12C show morphological changes of tau aggregation by d-isoprenaline observed with an atomic force microscope, FIG. 12A is a photograph showing aggregated tau without compound, control, FIG. 12B is a photograph showing an effect of d-isoprenaline on a tau aggregation sample, and FIG. 12C is a graph of measured major axes of tau aggregations and shows that d-isoprenaline reduces the numbers of granular and filamentous tau aggregations.

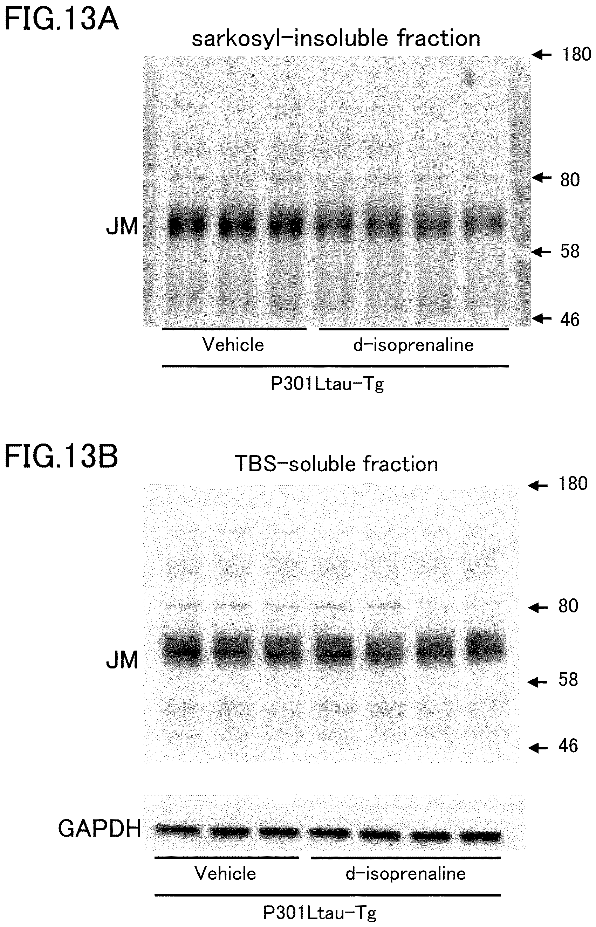

[FIG. 13] FIGS. 13A and 13B show a decrease in the amounts of sarkosyl-insoluble tau by d-isoprenaline in cerebral cortex of P301L tau Tg mice, FIG. 13A shows detection of tau in a sarkosyl-insoluble fraction, and FIG. 13B shows detection of tau and GAPDH (loading control) in a TBS-soluble fraction.

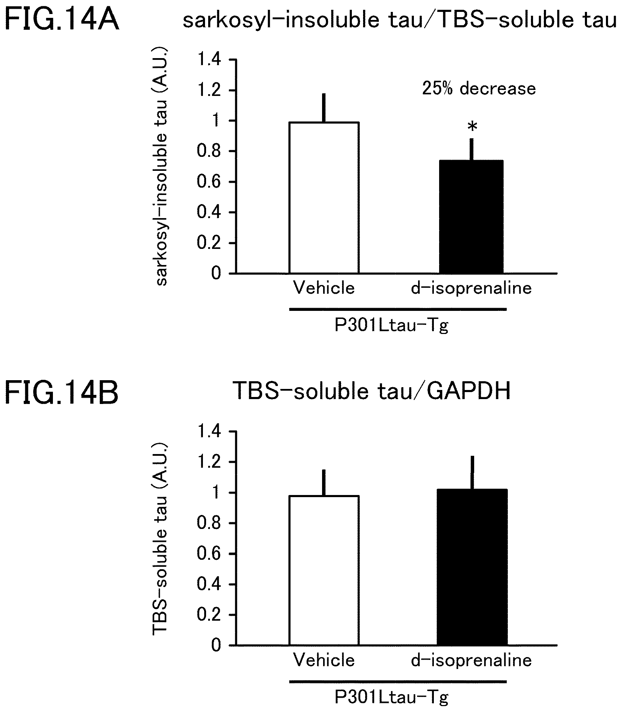

[FIG. 14] FIGS. 14A and 14B show a decrease in amount of sarkosyl-insoluble tau by d-isoprenaline in cerebral cortex of P301L tau Tg mice, FIG. 14A shows the proportion of sarkosyl-insoluble tau to TBS-soluble tau, and FIG. 14B shows the proportion of tau to GAPDH in a TBS-soluble fraction.

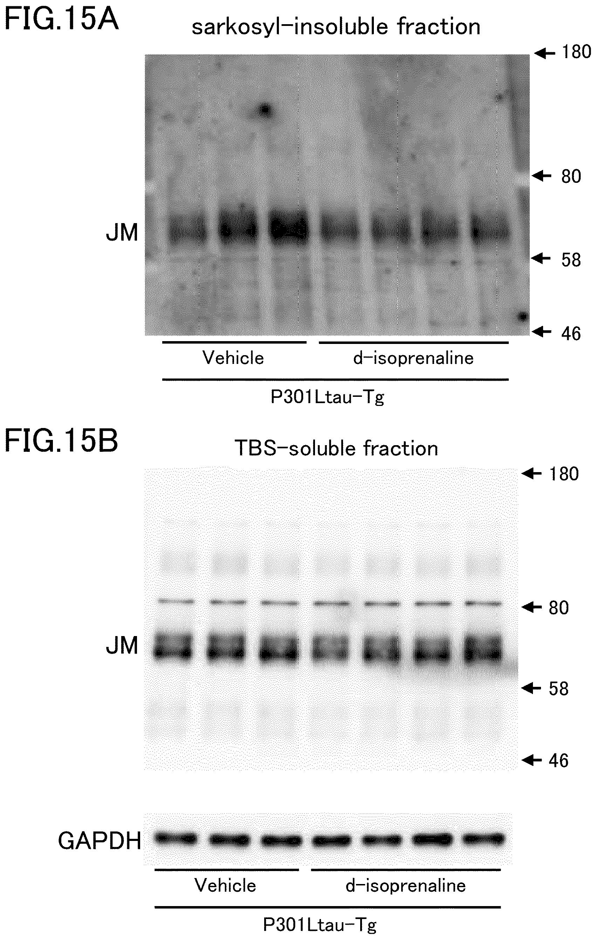

[FIG. 15] FIGS. 15A and 15B show a decrease in amount of sarkosyl-insoluble tau by d-isoprenaline in hippocampus of P301L tau Tg mice, FIG. 15A shows detection of tau in a sarkosyl-insoluble fraction, and FIG. 15B shows detection of tau and GAPDH (loading-control) in a TBS-soluble fraction.

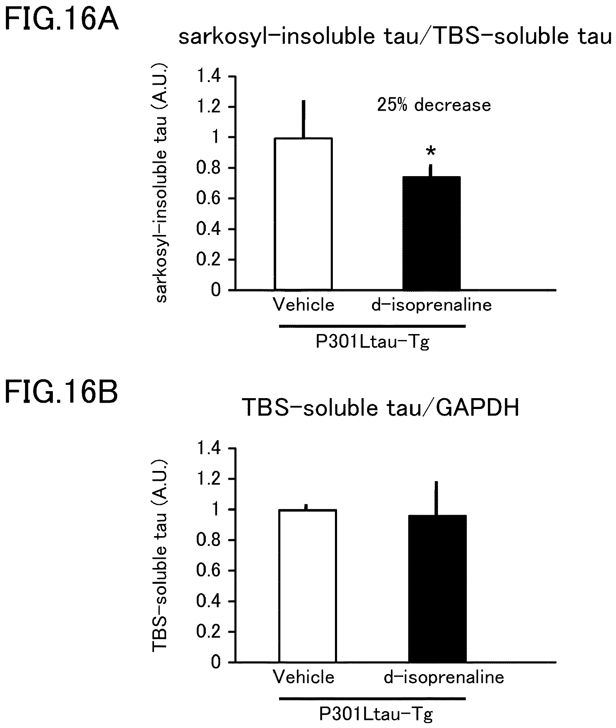

[FIG. 16] FIGS. 16A and 16B show a decrease in amount of sarkosyl-insoluble tau by d-isoprenaline in hippocampus of P301L tau Tg mice, FIG. 16A shows the proportion of sarkosyl-insoluble tau to TBS-soluble tau, and FIG. 16B shows the proportion of tau to GAPDH in a TBS-soluble fraction.

### DESCRIPTION OF EMBODIMENTS

An embodiment of the present invention will be specifically described with reference to the attached figures. The embodiment below is intended to facilitate understanding of the principle of the invention.

Inventors of the present invention have intensively investigated, to find for the first time that catechol structure-containing compounds are effective for prevention or therapy of tauopathies. Based on this finding, the inventors have achieved the invention. A catechol structure-containing compound herein refers to a compound containing a catechol structure in its constitutional formula. The catechol structure refers to a structure of catechol that is a compound in which two of its substituents are hydroxyl groups and these two hydroxyl groups are in ortho-positions each other.

The catechol structure-containing compound is droxidopa. A tau aggregation inhibitor according to the invention may be droxidopa alone or a combination of some or all of the catechol structure-containing compounds described above.

It is disclosed that Isoprenaline may be preferably used in any one of 1-enantiomer (R-configuration), d-enantiomer (S-configuration), or d/l-racemic mixture. Side effects of isoprenaline include palpitation and myocardial ischemia. In isoprenaline, l-enantiomer has greater effects than d-enantiomer, and side effects thereof decrease in the order of l-enantiomer, d/l-racemic mixture, and d-enantiomer. On the other hand, as described below, d-isoprenaline exhibits a tau aggregation inhibition effect to a degree similar to that of d/l-isoprenaline. Thus, between optical isomers, d-enantiomer is considered to be most preferable for use in a therapeutic agent for dementia because d-enantiomer has a similar tau aggregation inhibition effect but has smaller side effects than l-enantiomer.

Salts of these catechol structure-containing compounds are pharmacologically acceptable salts. Examples of the salts include: inorganic basic salts such as metal salts including alkali metal salts (e.g., potassium salt and sodium salt) and alkali-earth metal salts (e.g., magnesium salt and calcium salt), alkali metal carbonates (e.g., lithium carbonate, potassium carbonate, sodium carbonate, and cesium carbonate), alkali metal hydrogen carbonates (e.g., lithium hydrogen carbonate, sodium hydrogen carbonate, and potassium hydrogen carbonate), and alkali metal hydroxides (e.g., sodium hydroxide and potassium hydroxide); organic basic salts such as trialkylamine (e.g., trimethylamine and triethylamine), pyridine, quinoline, piperidine, imidazole, picoline, dimethylamino pyridine, dimethylaniline, N-alkyl-morpholine, DBN, and DBU; inorganic acid salts such as hydrochloric acid salt, hydrobromide, hydriodic acid salt, sulfate, nitrate, and phosphate; and organic acid salts such as formate, acetate, propionate, oxalate, malonate, succinate, fumarate, maleate, lactate, malate, citrate, tartrate, citrate, carbonate, picrate, methanesulfonate, and glutamate.

The tau aggregation inhibitor of this embodiment may contain an effective amount of droxidopa and salts thereof, together with a pharmacologically acceptable carrier. The carrier may be a solid such as an excipient or liquid such as a diluent. Specifically, examples of the carrier include magnesium stearate, lactose, starch, gelatin, agar, talc, pectin, gum arabic, olive oil, sesame oil, cacao butter, ethylene glycol, and distilled water.

Tauopathies are neurodegenerative diseases in which accumulation of phosphorylated tau occurs in neuronal cells and glia cells. Tauopathies are, for example, AD, Down's syndrome, Pick's disease, corticobasal degeneration (CBD), and progressive supranuclear palsy (PSP).

Prevention of tauopathies means preventing occurrence of tauopathy disorder. Therapy of tauopathies means preventing or improving/reducing progress of tauopathy disorder.

The tau aggregation inhibitor of this embodiment may contain, if necessary, one or more additives selected from the group consisting of pharmaceutically acceptable tonicity adjusting agents, buffers, solubilizers, preservatives, and pH adjusters.

Examples of the tonicity adjusting agents include potassium chloride, sodium chloride, boric acid, mannitol, glycerol, propylene glycol, polyethylene glycol, maltose, sucrose, sorbitol, and glucose.

Examples of the buffers include organic acids such as amino acid and succinic acid, inorganic acids such as boric acid and phosphoric acid, and pharmaceutically acceptable salts thereof.

Examples of the solubilizers include: polymers such as polyethylene glycol, polyvinylpyrrolidone, polyvinyl alcohol, and hydroxypropyl methylcellulose; surfactants such as polysorbate, polyoxyethylene hydrogenated castor oil, and polyoxyethylene polyoxypropylene; polyhydric alcohol such as propylene glycol; organic acids such as benzoic acid and sorbic acid; and amino acids such as aspartic acid, histidine, glycine, and lysine.

Examples of the preservatives include: quaternary ammonium salts such as benzethonium, benzalkonium, and benzododecinium; cation compound salts such as chlorhexidine; parahydroxybenzoic acid esters such as methyl parahydroxybenzoate and propyl parahydroxybenzoate; and alcohol compounds such as chlorobutanol and benzyl alcohol.

Examples of the pH adjusters include sulfuric acid, hydrochloric acid, acetic acid, lactic acid, calcium hydroxide, potassium hydroxide, sodium hydroxide, magnesium hydroxide, monoethanolamine, triethanolamine, diisopropanolamine, and triisopropanolamine.

The dose of the tau aggregation inhibitor of this embodiment is not specifically limited as long as appropriate effects are obtained, and is determined in consideration of the degree of symptoms, sexuality, and ages of patients to be treated. For example, the dose of the tau aggregation inhibitor can be 0.0001 to 1000 mg per day for an adult. This dose of the inhibitor per day may be administered once daily or may be divided for several administrations daily.

The tau aggregation inhibitor of this embodiment can be prepared in formulation types in accordance with the manner of administration. Examples of oral administration types include solid formulations and solution formulations including granules, balls, tablets, capsules, powders, and solutions. Examples of parenteral administration types include injections such as intravenous injection and intramuscular injection.

When phosphorylated, tau-tau association occurs and tau oligomers are formed. When these tau oligomers grow to have a beta-pleated sheet structure, spherical granular tau aggregation is formed. The granular tau aggregation is considered to be constituted by about 40 tau molecules. The granular tau aggregations are joined together to form neurofibrillary tangles (NFTs) called paired helical filaments (PHFs). Recent researches using mouse models shows that inhibition of tau overexpression in the period of NFT formation improves memory learning of mice but formation of NFTs continues. This suggests that neuronal dysfunction occurs mainly in the process of formation of NFTs, rather than being caused by NFTs themselves. NFTs themselves are not toxic, and products formed in the process of NFT formation is considered to be a major cause of neurotoxicity. The tau aggregation inhibitor of this embodiment inhibits not only tau aggregation in the process of PHF formation by joined granular tau aggregation but also tau aggregation in the process of formation of spherical granular tau aggregations. Neurodegeneration in brain occurs by not only accumulation of mutant tau protein but also accumulation of wild-type tau. The tau aggregation inhibitor of the present invention can inhibit aggregation of wild-type tau. Thus, tauopathy symptoms including AD can be prevented or treated.

### [Examples]

### (Example 1)

Compounds that can be bound to tau were screened. First, 10 µM of 2N4R tau (TAU-441 HUMAN) and 10 µM of heparin were mixed together and incubated at 37°C to form tau aggregates. This aggregated tau sample (1 ml) was loaded onto a sucrose-density gradient solution (consisting of 1 ml layers 20%, 30%, 40%, and 50%), and was centrifuged (at 200000 xg for 2 h at 20°C). Then, the solution was collected in units of 1 ml from the top layer to obtain samples of fractions (Fr) 1-5. The resultant pellets were suspended in an HEPES solution to obtain a fraction Fr6. Thereafter, binding capacities between tau included in Fr 1, 3, and 5 with predetermined 6600 compounds were analyzed by a surface plasmon resonance technique. The surface plasmon resonance technique is a technique for analyzing an intermolecular interaction between two molecules by monitoring a change in refractive index caused by, for example, a change in molecular mass fixed on a thin gold film. The surface plasmon resonance technique can be performed with a commercially available surface plasmon resonance system, e.g., BIAcore 2000 (produced by Pharmacia Biosensor). As a result, it was found that 111 compounds out of the 6600 compounds were bound to tau.

Then, it was analyzed, by thioflavine T stain, whether the 111 compounds inhibit tau aggregation. For this analysis, 10-µM tau, a compound (1 µM, 10 µM, or 100 µM), and thioflavine T (a beta-pleated sheet structure-specific detection reagent) were mixed together. Thereafter, heparin, which is a tau aggregation inducer, was added to the mixture, and the resulting mixture was incubated at 37°C, thereby allowing tau to aggregate. Subsequently, the thioflavine T activity in an incubation sample was measured at various times to investigate tau aggregation inhibition effects by the compounds. As a result, it was observed that nine out of the 111 compounds noticeably inhibited the thioflavine T activity in a low concentration of 1 µM.

To further investigate the effects of these nine compounds on tau aggregation specifically, samples that had been incubated at 37°C were centrifuged at 250000 xg for 2 h, the resultant pellets were obtained so that the amount of insoluble tau were quantified. As a result, it was found that (R)-(-)-epinephrine and pyrocatechol violet reduced the amount of insoluble tau in a concentration dependent manner. These two compounds, i.e., (R)-(-)-epinephrine and pyrocatechol violet, were found to have the same skeleton of catechol nucleus.

### (Example 2)

Then, it was analyzed, by thioflavine T stain, whether compounds whose structures resemble to (R)-(-)-epinephrine and pyrocatechol violet inhibit tau aggregation. As a result, as shown in FIG. 1, levodopa, dopamine, norepinephrine, and isoprenaline significantly reduced thioflavine T activity, similar to (R)-(-)-epinephrine. FIG. 1A shows a change in thioflavine T activity by (R)-(-)-epinephrine, FIG. 1B shows a change in thioflavine T activity by levodopa, FIG. 1C shows a change in thioflavine T activity by dopamine, FIG. 1D shows a change in thioflavine T activity by norepinephrine, and FIG. IE shows a change in thioflavine T activity by isoprenaline.

Among these samples, the (R)-(-)-epinephrine sample and the isoprenaline samples were divided into fractions Fr1-Fr6 by sucrose-density gradient centrifugation, and tau was detected by SDS-PAGE western blotting. The concentration of the compounds used was 100 µM. As a result, as shown in FIG. 2, aggregated tau was detected in fractions Fr3, 4, 5, and 6 in Control, whereas 100-µM of compounds reduced the amount of tau in fractions Fr3, 4, 5, and 6. FIG. 2A shows tau aggregation inhibition effects of (R)-(-)-epinephrine, and FIG. 2B shows tau aggregation inhibition effects of isoprenaline. From the foregoing results, a novel common structure that inhibits tau aggregation *in vitro* was found. Among these compounds, isoprenaline is an existing drug, and is considered to have higher extents of safety than other drugs of catecholamines. Thus, isoprenaline was used in the following analyses.

### (Example 3)

Then, it was investigated whether isoprenaline inhibits tau aggregation in cultured cells. As cells, Neuro2a cell lines in which human P301L mutant tau (i.e., mutant tau in which 301st proline of tau is changed to leucine) is expressed in stable were used. To these cells, isoprenaline was added in concentrations of 0.01, 0.1, and 1 µM for 48 hours. Then, SDS-insoluble fractions were obtained so that a change in the amounts of tau was observed. As a result, as shown in FIG. 3, isoprenaline reduced the amounts of SDS-insoluble tau similarly to lithium chloride, a positive control, which is a glycogen synthase kinase 3β (GSK3β) inhibitor (e.g., a material that physically or chemically inhibits the function of GSK3β). FIG. 3A shows detection of tau in SDS-insoluble fractions, and FIG. 3B is a graph corresponding to FIG. 3A, and shows that isoprenaline reduces the amount of SDS-insoluble tau.

In addition, changes of tau phosphorylation in radio-immunoprecipitation assay (RIPA) buffer-soluble fractions obtained from similar cells were analyzed. As a result, as shown in FIG. 4, it was found that isoprenaline reduced tau phosphorylation labeled by anti-phosphorylated tau antibody (AT8). The composition of the RIPA buffer was 50mM Tris-HCl (pH7.4), 150mM sodium chloride, 0.25 w/v% sodium deoxycholate, 1mM EGTA, and 1.0 w/v% NP-40 substitute. Thus, it was found that isoprenaline also inhibits tau phosphorylation as well as tau aggregation. FIG. 4A shows detection of phosphorylated tau (AT8 site) and total tau in RIPA-soluble fractions, and FIG. 4B shows proportions of phosphorylated tau to total tau.

### (Example 4)

Tau phosphorylations at AT8 sites are known to induce a tau conformational change (that can be detected with the MC1 antibody) observed in AD brains. Thus, WT tau was expressed in COS-7 cells (derived from African green monkey kidney), and a tau conformational change was detected by dot blotting using the MC1 antibody. The dot blotting is a technique of fixing protein to a nitrocellulose membrane or a PVDF membrane without separation through electrophoresis and specifically quantitating the protein amount with enzyme-labeled antibodies. As a result, as shown in FIGS. 5A and 5B, isoprenaline reduced the tau conformational change detected with the MC1 antibody. FIG. 5A shows detections of MC1 antibody-positive tau, tau phosphorylations (AT8 sites), total tau, and GAPDH (loading control) in a TBS-soluble fraction. FIG. 5B shows the proportion of MC1 antibody-positive tau with respect to total tau. As shown in FIG. 5C, isoprenaline also reduced phosphorylation under similar conditions. FIG. 5C shows the proportion of phosphorylated tau with respect to total tau.

### (Example 5)

It was investigated how isoprenaline affects binding between microtubules and tau. First, 10 µM of isoprenaline was added to COS-7 cells expressing WT tau, and the cells were left for 24 hours and then homogenized with RA buffer (0.1-µM MES, 0.5-mM MgSO₄, 1-mM EGTA, 2-mM DTT, 0.1% TritonX-100, 20-µM taxol, and 2-mM GTP). The homogenates were then centrifuged at 3000 xg for 5 min at 25°C, and the a supernatant was obtained as a total fraction. The total fraction was further centrifuged at 100000 xg for 20 min at 20°C, and a pellet (a microtubule fraction) was obtained. In this manner, the microtubule fraction was taken from the COS-7 cells expressing WT tau, and acetylated tubulin serving as an index of tau and microtubule stabilization was detected.

As a result, as shown in FIG. 6, in WT tau-expressing COS-7 cells, 10-µM isoprenaline increased the amount of tau in the microtubule fraction. FIG. 6A shows detection of tau in the microtubule fraction. FIG. 6B shows detection of tau in the total fraction. FIG. 6C is a graph corresponding to FIG. 6A.

In the WT tau-expressing COS-7 cells, the amount of acetylated tubulin increased as compared to vector-expressing cells. Addition of isoprenaline to these cells further increased the amount of acetylated tubulin, as shown in FIGS. 6D, 6E, and 6F. Thus, the results suggest the possibility that isoprenaline stabilizes of microtubules by increasing the amount of tau bound to the microtubules. FIG. 6D shows acetylated tubulin in of the microtubule fraction. FIG. 6E shows acetylated tubulin in of the total fraction. FIG. 6F is a graph corresponding to FIG. 6D.

### (Example 6)

It was investigated whether isoprenaline inhibits tau aggregation in mice. First, P301L tau Tg mice were given isoprenaline (1.5 mg/g fed) mixed in mash for three months. Then, cerebral cortex and hippocampus were excised from the mice, and stored at -80°C. To obtain fractions including soluble tau and insoluble tau from these tissues, a frozen tissues were homogenized in TBS solution, centrifuged (at 23000 rpm for 15 min at 4°C), and then fractionated into the supernatant and the a pellet. The supernatant was used as a TBS-soluble fraction (including soluble tau). In addition, 0.32M of sucrose was added to the pellet, and the pellet was homogenized again, centrifuged (at 23000 rpm for 15 min at 4°C), and then fractionated into the supernatant (including tau aggregation) and the a pellet (including nuclei). Thereafter, surfactant (1% sarkosyl) was added to the supernatant, and the resulting supernatant was incubated (at 37°C for 1 h) and centrifuged (at 200000 xg for 1 h at 4°C). A pellet dissolved with Laemmli buffer (containing 2-mercaptoethanol) was used as a sarkosyl-insoluble fraction. Tau in the TBS-soluble fraction and the sarkosyl-insoluble fraction was detected by SDS-PAGE western blotting. As a result, as shown in FIG. 7, isoprenaline reduced the amount of sarkosyl-insoluble tau in the brains of the P301L tau Tg mice. FIG. 7A shows detection of tau in the sarkosyl-insoluble fraction, and FIG. 7B is a graph in which the result of FIG. 7A is quantitated. In the graph, Ntg means Non-transgenic mice.

### (Example 7)

In the brains of P301L tau Tg mice, the numbers of neurons are decreased accompanying tau aggregates formation. Thus, there is the possibility that isoprenaline having a tau aggregation inhibition function can suppress decreases in the number of neuronal cells. Thus, brain slices were prepared from isoprenaline-administered mice, and the number of neuronal cells was counted. To measure the number of neuronal cells, 0.1-mm² boxes as shown in FIG. 8A were drawn in entorhinal cortex or temporal area, and the number of cells in each of the boxes was counted. Then, the average number of cells was used as a number for one slice. Two slices were prepared from one individual. As a result, as shown in FIGS. 8B and 8C, isoprenaline suppressed a decrease in number of cells in entoehinal cortex and temporal area as indicated in the P301L tau Tg mice. This suggests that isoprenaline can suppress a decrease in number of neuronal cells by inhibiting tau aggregation. In FIGS. 8B and 8C, "Mice" refers to the number of mice used, and "Slice" refers to the number of slices per one individual in which the number of cells was counted.

### (Example 8)

A change in tau phosphorylation in TBS-soluble fractions from WT tau Tg mice was investigated. As a result, as shown in FIG. 9, isoprenaline induced dephosphorylation of tau labeled by monoclonal antibody tau1. FIG. 9A shows a TBS-soluble fraction, and FIG. 9B shows the proportion of dephosphorylated tau to tau. The results suggest that isoprenaline also inhibits tau aggregation and phosphorylation of total tau in mice.

The above examples show effects of isoprenaline. However, since a catechol amine moiety was found as a novel common structure that inhibits tau aggregation (see Example 2) and dopamine, dobutamine, levodopa, levodopa/carbidopa, trimetoquinol, hexoprenaline, methyldopa, and droxidopa have catechol amine structures similar to that of isoprenaline, these materials appear to have the effect of inhibiting tau aggregation in cultured cells and animals, similarly to isoprenaline.

### (Example 9)

Tau (10 µM), d- and d/l-isoprenaline (1-100 µM), and thioflavine T were mixed together. Then, heparin was added to the mixture, and the resulting mixture was incubated at 37°C, thereby allowing tau to aggregate. In the period shown in the figures, the thioflavine T activity in incubation samples were analyzed to investigate tau aggregation inhibition effects of compounds. FIG. 10A is a graph showing the effect of d-isoprenaline on the thioflavine T activity. FIG. 10B is a graph showing the effect of d/l-isoprenaline on the thioflavine T activity. As shown in FIGS. 10A and 10B, d-isoprenaline exhibited an inhibition of thioflavine T activity similar to that of d/l-isoprenaline.

Then, to analyze a change of tau aggregation by d- and d/l-isoprenaline biochemically, an incubated tau aggregation sample was fractionated into Fr1-Fr6 by sucrose-density gradient centrifugation, and tau was detected by SDS-PAGE western blotting. FIG. 11 shows results of SDS-PAGE western blotting indicating tau aggregation inhibition effects detected by sucrose-density gradient centrifugation. FIG. 11A shows results of d-isoprenaline, and FIG. 11B shows results of d/1-isoprenaline. As shown in FIGS. 11A and 11B, similarly to d/l-isoprenaline, d-isoprenaline apparently reduced the amount of tau (granular and filamentous tau aggregates) fractionated into Fr3 and subsequent fractions.

Thereafter, to analyze a change of tau aggregation by d-isoprenaline morphologically, the sample was investigated by using an atomic force microscope. A tau aggregation sample that had been incubated for 120 hours was loaded on a mica board and adsorbed thereon. After removal of the sample, the mica board was filled with milliQ water, and the aggregated tau was observed with an atomic force microscope. FIG. 12 shows morphological change of tau aggregation observed with an atomic force microscope. FIG. 12A is a photograph of a control, FIG. 12B is a photograph showing effects of d-isoprenaline on a tau aggregation sample, and FIG. 12C is a graph of measured major axes of tau aggregation. As shown in FIGS. 12A, 12B, and 12C, d-isoprenaline apparently reduced the number of tau aggregates (granular and filamentous tau aggregates) having major axes of 20 nm or more.

To investigate tau aggregation inhibition effects of d-isoprenaline *in vivo* P301L tau Tg mice were used for analysis. First, P301L tau Tg mice aged 20-21 months were given d-isoprenaline (2.168 mg/g fed) mixed in mash for three months. Then, cerebral cortices and hippocampi were excised from the mice and stored at -80°C. Thereafter, a TBS-soluble fraction and a sarkosyl-insoluble fraction were prepared from these brain tissues, and tau was analyzed by SDS-PAGE western blotting.

FIG. 13 shows decreases in amount of sarkosyl-insoluble tau in cerebral cortices of d-isoprenaline-treated P301L tau Tg mice. FIG. 13A shows detection of tau in the sarkosyl-insoluble fraction. FIG. 13B shows detection of tau in the TBS-soluble fraction and GAPDH in a loading control. FIG. 14 shows decreases in amount of sarkosyl-insoluble tau by d-isoprenaline in cerebral cortex of P301L tau Tg mice. FIG. 14A shows the proportion of sarkosyl-insoluble tau to TBS-soluble tau. FIG. 14B shows the proportion of tau to GAPDH in the TBS-soluble fraction. As shown in FIGS. 13 and 14, d-isoprenaline apparently reduced the amount of insoluble tau in cerebral cortices of P301L tau Tg mice.

In a manner similar to the above-described examination of the amount of insoluble tau in cerebral cortex of P301L tau Tg mice, the amount of insoluble tau in hippocampus of P301L tau Tg mice were obtained. FIG. 15 shows decreases in amount of sarkosyl-insoluble tau by d-isoprenaline in hippocampus of P301L tau Tg mice. FIG. 15A shows detection of tau in a sarkosyl-insoluble fraction. FIG. 15B shows detection of tau in a TBS-soluble fraction and GAPDH in a loading control. FIG. 16 shows decreases in amount of sarkosyl-insoluble tau by d-isoprenaline in hippocampus of P301L tau Tg mice. FIG. 16A shows the proportion of sarkosyl-insoluble tau to TBS-soluble tau. FIG. 16B shows the proportion of tau to GAPDH in the TBS-soluble fraction. As shown in FIGS. 15 and 16, d-isoprenaline apparently reduced the amount of insoluble tau in hippocampi of P301L tau Tg mice.

### INDUSTRIAL APPLICABILITY

The present invention is useful for treatment of tauopathies.

## Claims

1. A tau aggregation inhibitor for use in prevention and/or treatment of tauopathy, wherein
the tau aggregation inhibitor includes a catechol structure-containing compound or a salt thereof, and
the catechol structure-containing compound is droxidopa;
wherein the tauopathy excludes Alzheimer's disease.

## Patentansprüche

1. Tau-Aggregationshemmer zur Verwendung in der Vorbeugung und/oder Behandlung von Tauopathie, wobei
der Tau-Aggregationshemmer eine Catecholstruktur-haltige Verbindung oder ein Salz davon einschließt, und
die Catecholstruktur-haltige Verbindung Droxidopa ist;
wobei die Tauopathie Alzheimer-Erkrankung ausschließt.

## Revendications

1. Inhibiteur d'agrégation de tau pour une utilisation dans la prévention et/ou le traitement de la tauopathie, où
l'inhibiteur d'agrégation de tau comporte un composé contenant une structure de catéchol ou un sel de celui-ci, et
le composé contenant une structure de catéchol est la droxidopa ;
où la tauopathie exclut la maladie d'Alzheimer.
